# EUROPÄISCHE PATENTSCHRIFT

(11) **EP 3 062 530 B1**
(45) Veröffentlichungstag und Bekanntmachung des Hinweises auf die Patenterteilung: **03.04.2019**
(21) Anmeldenummer: 16156415.8
(22) Anmeldetag: 19.02.2016
(51) Int. Cl.: H04R 25/00, A61B 5/12, H04R 1/10

(54) **VORRICHTUNG ZUR ERMITTLUNG EINER PERSÖNLICHEN TINNITUS-FREQUENZ**
DEVICE FOR DETERMINING A PERSONAL TINNITUS FREQUENCY
DISPOSITIF DE DETERMINATION D'UNE FREQUENCE D'ACOUPHENE PERSONNELLE

(30) Priorität: 27.02.2015 DE 102015102875
(43) Veröffentlichungstag der Anmeldung: 31.08.2016
(73) Patentinhaber: Resaphene Suisse AG, 9325 Roggwil, TG (CH)
(72) Erfinder: Merz, Volker, 78647 Trossingen (DE)
(74) Vertreter: Otten, Roth, Dobler & Partner mbB Patentanwälte

(56) Entgegenhaltungen:
- CN-U- 202 682 155
- DE-A1-102010 039 589
- DE-A1-102012 220 620
- US-A- 2 344 023
- US-A- 4 513 197
- US-A1- 2011 051 947
- US-A1- 2013 131 542
- US-A1- 2014 126 733
- US-A1- 2015 038 774
- US-B1- 7 377 666

## Beschreibung

Die Erfindung betrifft eine Vorrichtung zur Ermittlung einer persönlichen Tinnitus-Frequenz, bestehend aus einer Audiosignal-Erzeugereinheit und einem zugeordneten Audiosignal-Kopfhörer, welche ein in der Frequenz stufenlos einstellbares erstes Audiosignal bereitstellt.

### Stand der Technik

Vorrichtungen zur Ermittlung einer persönlichen Tinnitus-Frequenz sind bekannt und werden beispielsweise in Arztpraxen in speziell dafür vorgesehenen Räumen, welche gegenüber Umweltgeräuschen akustisch isoliert sind, also in einer stillen Umgebung zur Ermittlung eines persönlichen Tinnitus verwendet. Tinnitus-Ermittlungs oder -Therapiegeräte sind z.B. aus der DE 10 2010 039 589 A1 und der US 2013/0131542 A1 bekannt.

Ein Nachteil dieser Vorrichtungen sind für den Nutzer der Weg und die damit verbundene Zeit zum und beim Arzt. Ein weitere Nachteil sind die aufgrund einer mangelhaften akustischen Isolation in den Behandlungsräumen des Arztes auftretenden Umweltgeräusche, wie beispielsweise ein Telefonklingeln, welche eine Ermittlung und Behandlung eines persönlichen Tinnitus mit bekannten Vorrichtungen erschweren.

### Aufgabe und Vorteile der Erfindung

Der Erfindung liegt die Aufgabe zugrunde, eine verbesserte Vorrichtung, zur Ermittlung und Therapierung eines persönlichen Tinnitus, zur Verwendung durch den Tinnitus-Geschädigten, bereitzustellen.

Diese Aufgabe wird durch die Merkmale des Anspruchs 1 gelöst. In den abhängigen Ansprüchen sind vorteilhafte und zweckmäßige Ausführungsformen der Erfindung angegeben.

Die Erfindung geht von einer Vorrichtung zur Ermittlung einer persönlichen Tinnitus-Frequenz aus, bestehend aus einer Audiosignal-Erzeugereinheit und einem zugeordneten Audiosignal-Kopfhörer, welcher ein in der Frequenz stufenlos einstellbares erstes Audiosignal bereitstellt.

Ein vorteilhafter Aspekt der Erfindung ist nun darin zu sehen, dass Kompensationsmittel vorgesehen sind, um Umweltgeräusche durch eine aktive Geräuschunterdrückung zu eliminieren.

Ein zugeordneter Audiosignal-Kopfhörer kann beispielsweise über eine Kabelverbindung oder auch über eine Funkverbindung mit der Audiosignal-Erzeugereinheit verbunden sein.

Beispielsweise ist an einen Audiosignal-Kopfhörer eine Energieversorgungseinheit, zum Beispiel einer Batterie oder einem Akku angeordnet.

Ein Kompensationsmittel zur aktiven Unterdrückung von Umweltgeräuschen kann insbesondere an einem Audiosignal-Kopfhörer angeordnet sein, welcher beispielsweise als sogenannter Anti-Noise-Cancelling-Kopfhörer (ANC-Kopfhörer) ausgestaltet ist.

Durch die aktive Unterdrückung von Umweltgeräuschen ist das Tinnitus-Therapiegerät in einer gewöhnlichen, häuslichen Umgebung als Wellnessgerät zur Therapierung eines Tinnitus einsetzbar.

Ein Nutzer kann zum Beispiel durch Einstellmittel, welche an der Vorrichtung angeordnet sind, eine Frequenz und/oder eine Phase der Frequenz und/oder gegebenenfalls eine Amplitude eines eingespielten ersten Audiosignals stufenlos verändern und seine persönliche Tinnitus-Frequenz und gegebenenfalls die Phase der persönlichen Tinnitus-Frequenz und möglicherweise die Amplitude der persönlichen Tinnitus-Frequenz in häuslicher Umgebung dadurch selbst bestimmen.

Unter Einstellmittel werden unter anderem beispielsweise Drehregler, Schieberegler, Regelknöpfe, Drehknöpfe, Kippschalter, Druckschalter aber auch berührungslose Bedienelemente, welche zum Beispiel durch Gesten, insbesondere durch Sprache bedient werden, verstanden.

Weiterhin denkbar ist, dass eine Frequenz und/oder eine Phase der Frequenz und/oder eine Amplitude eines eingespielten ersten Audiosignals für jedes Ohr getrennt über ein Einstellmittel an der Vorrichtung zur Ermittlung einer persönlichen Tinnitus-Frequenz einstellbar ist.

Die ermittelte persönliche Tinnitus-Frequenz und gegebenenfalls die ermittelte Phase der persönlichen Tinnitus-Frequenz und möglicherweise die Amplitude der persönlichen Tinnitus-Frequenz kann an der Vorrichtung zur Ermittlung einer persönlichen Tinnitus-Frequenz abgelegt werden. Beispielsweise auf einer nicht weiter definierten Speichereinheit, welche an der Vorrichtung zur Ermittlung einer persönlichen Tinnitus-Frequenz angeordneten ist. Mittels außerdem angeordneter Einstellmittel kann die Information insbesondere wiederaufrufbar abgelegt werden.

Vorstellbar ist auch, dass ein Nutzer eine zuvor anderweitig und/oder zu einem anderen Zeitpunkt und/oder an einem anderen Ort und/oder mit einer anderen Vorrichtung ermittelte persönliche Tinnitus-Frequenz und/oder gegebenenfalls die ermittelte Phase der persönlichen Tinnitus-Frequenz und/oder möglicherweise die Amplitude der persönlichen Tinnitus-Frequenz an der Vorrichtung zur Ermittlung einer persönlichen Tinnitus-Frequenz auf einer nicht weiter definierten Speichereinheit wiederaufrufbar ablegen kann.

Der wesentliche Aspekt der Erfindung liegt darin, dass eine Heizanordnung zur Erwärmung eines Ohrbereichs an der Vorrichtung vorgesehen ist. Die Heizanordnung ist zusätzlich zur oben beschriebenen Vorrichtung oder unabhängig von einer Geräuschunterdrückung einsetzbar.

Die Heizanordnung ist an einem Audiosignal-Kopfhörer angeordnet. Der beheizbare Audiosignal-Kopfhörer wird dazu eingesetzt, die Durchblutung des Ohres, beispielsweise des Mittel- und/oder Innenohres zu stimulieren.

Der beheizbare Audiosignal-Kopfhörer und/oder die Vorrichtung zur Ermittlung einer persönlichen Tinnitus-Frequenz kann, zum Beispiel, derart ausgestaltet sein, dass eine Temperatur für jedes Ohr getrennt stufenlos regelbar einstellbar ist. An der Vorrichtung zur Ermittlung einer persönlichen Tinnitus-Frequenz ist ein Einstellmittel zur Einstellung einer Temperatur eines beheizbaren Kopfhörers angeordnet.

Der Audiosignal-Kopfhörer ist als Ohrstöpsel ausgebildet.

Indem ein Audiosignal-Kopfhörer als Ohrstöpsel ausgebildet ist, welcher als In-Ear-Kopfhörer bezeichnet wird, kann der Tragekomfort des Audiosignal-Kopfhörers verbessert werden.

Die Ausbildung eines Audiosignal-Kopfhörers als Ohrstöpsel ermöglicht eine individuellen Ohrstöpselgestaltung an eine Ohr- bzw. Gehörganggeometrie des Nutzers und somit eine vergleichsweise Verbesserung des Tragekomforts. Zum Beispiel kann der Ohrstöpsel ein Ohrstöpsel-Hüllelement aufweisen, welches den Ohrstöpsel des Audiosignal-Kopfhörers umschließt und an die individuelle Ohr- bzw. Gehörganggeometrie des Nutzers angepasst ist.

Vorteilhafterweise kann das Ohrstöpsel-Hüllelement aus Silikon ausgebildet sein. Silikon kann insbesondere Wärme vergleichsweise gut leiten, dadurch kann es sich beispielsweise gut als Material für den Einsatz an einem Audiosignal-Kopfhörer mit Heizanordnung eignen. Dadurch kann einfach das Ohr beziehungsweise der Gehörgang des Ohrs erwärmt werden.

Ein Audiosignal-Kopfhörer, welcher als Ohrstöpsel ausgebildet ist, kann beispielsweise sowohl das Innen- als auch das Mittelohr getrennt voneinander mit unterschiedlichen Temperaturen erwärmen.

Weiter wird vorgeschlagen, dass über ein an die Audiosignal-Erzeugereinheit angeordnetes Einstellmittel ein erstes und/oder zweites Audiosignal auswählbar ist.

Ein Nutzer kann durch Einstellmittel, welche an der Vorrichtung angeordnet sind, beispielsweise die Möglichkeit haben am Tinnitus-Therapiegerät ein erstes Audiosignal auszuwählen. Das erste Audiosignal kann derart ausgebildet sein, dass es einem persönlichen Tinnitus-Ton, zum Beispiel einem Piep-Ton, einem Klingel-Ton, einem Rauschen, oder einem anderweitig ausgebildeten Tinnitus-Ton am nächsten kommt.

Das Geräusch kann sich beispielsweise auch aus mehreren Frequenzen zusammensetzen. Verändert der Nutzer an der Vorrichtung zur Ermittlung einer persönlichen Tinnitus-Frequenz die einstellbare Frequenz mittels eines Einstellmittels, um seine persönliche Tinnitus-Frequenz zu ermitteln, verändert das Tinnitus-Therapiegerät alle im ersten Audiosignal auftretenden Frequenzen, mittels einem an der Audiosignal-Erzeugereinheit angeordnetem Einstellmittel, entsprechend. Beispielsweise werden alle im ersten Audiosignal auftretenden Frequenzen um +1 Hz verändert, wenn der Nutzer die einstellbare Frequenz mittels des Einstellmittels der Audiosignal-Erzeugereinheit um +1 Hz verändert.

Es ist überdies vorteilhaft, dass die Vorrichtung ein Element aufweist, mit welchem eine ermittelte persönliche Tinnitus-Frequenz in einem schmalen Frequenzbereich stufenlos regelbar ist.

Beispielsweise weist das Tinnitus-Therapiegerät ein Einstellmittel auf, mit welchem man die ermittelte persönliche Tinnitus-Frequenz in einem schmalen Frequenzbereich um die persönliche Tinnitus-Frequenz herum stufenlos verändern kann.

Weiter wird vorgeschlagen, dass die Vorrichtung dazu ausgebildet ist, ein zweites Audiosignal zu erzeugen, in welchem eine ermittelte persönliche Tinnitus-Frequenz verändert ist.

Ein auf einem Audiosignal-Kopfhörer einspielbares zweites Audiosignal, welches in einer nicht näher definierten Speichereinheit der Vorrichtung zur Ermittlung einer persönlichen Tinnitus-Frequenz oder einer extern Speichereinheit wiederaufrufbar abgelegt und auswählbar ist, kann mittels eines Audioelements, welches an der Audiosignal-Erzeugereinheit angeordnet ist, derart verändert werden, dass eine ermittelte persönliche Tinnitus-Frequenz verändert ist.

Das zweite Audiosignal kann beispielsweise ein Ton, ein Geräusch, oder auch eine Musik beziehungsweise eine Melodie umfassen.

Eine ermittelte persönliche Tinnitus-Frequenz kann beispielsweise aus dem zweiten Audiosignal durch ein Audioelement herausgefiltert werden, zum Beispiel enthält das zweite Audiosignal nur Frequenzen welche im ermittelten persönlichen Tinnitus-Ton nicht enthalten sind.

Außerdem vorstellbar ist, dass das zweite Audiosignal dem ersten Audiosignal durch ein Audioelement überlagert wird.

Hierdurch kann ein Nutzer z.B. ein angenehmes zweites Audiosignal, beispielsweise (Entspannungs-)Musik oder angenehme Geräusche (Vogelgezwitscher, Bachrauschen), wahrnehmen und unbewusst das vergleichsweise unangenehme erste Audiosignal. Dies kann eine Konditionierung des Nutzers dahingehend ermöglichen, dass der Nutzer im Alltag seinen unangenehmen Tinnitus-Ton verdrängt bzw. nicht unangenehm wahrnimmt.

Denkbar ist auch, dass durch ein Audioelement, an der Stelle oder im Bereich einer ermittelten persönlichen Tinnitus-Frequenz im zweiten Audiosignal, im Bezug zu einer definierten Phase einer ermittelten persönlichen Tinnitus-Frequenz in einem ersten Audiosignal, eine Phasenverschiebung vorgenommen wird.

Beispielsweise kann, in einem Modus des zweiten Audiosignals, die Phase bei der Tinnitus-Frequenz des zweiten Audiosignals, genau um 180° phasen-verschoben, zur definierten Phase der persönlichen Tinnitus-Frequenz des ersten Audiosignals, sein. Überlagert sich die Phasen destruktiv können sie sich auslöschen. Bei weniger als 180° Phasenverschiebung findet gegebenenfalls eine Teilauslöschung statt.

Vorstellbar ist auch, dass in einem weiteren Modus des zweiten Audiosignals, die beiden Phasen beispielsweise genau in Phase sind und die persönliche Tinnitus-Frequenz bzw. der persönliche Tinnitus-Frequenzbereich somit konstruktiv überlagert wird und sich verstärkt.

Außerdem kann, in einem weiteren Modus des zweiten Audiosignals, die Phase der ermittelten persönlichen Tinnitus-Frequenz im zweiten Audiosignal über ein Einstellmittel an der Vorrichtung zur Ermittlung einer persönlichen Tinnitus-Frequenz stufenlos veränderbar sein.

Insbesondere kann es denkbar sein, dass Frequenzen in einem zweiten Audiosignal, in einem weiteren Modus des zweiten Audiosignals, in einem schmalbandigen Bereich um die ermittelte persönliche Tinnitus-Frequenz über ein Einstellmittel an der Vorrichtung veränderbar sind.

Überdies von Vorteil ist, dass an die Audiosignal-Erzeugereinheit ein Element angeordnet ist, an welches eine Speichereinheit angeschlossen werden kann.

Die Speichereinheit kann beispielsweise Audiodaten für das erste und/oder das zweite Audiosignal bereitstellen.

Außerdem kann die Speichereinheit beispielsweise als Stick, als Festplatte, oder als Speicherkarte ausgebildet sein. Das Element kann beispielsweise eine USB- und/oder Bluetooth- und/oder NFC- und/oder WLAN-Schnittstelle und/oder eine Schnittstelle, mit welcher verschiedene Speicherkartentypen gelesen werden können umfassen.

Auch ist es von Vorteil, dass die Audiosignal-Erzeugereinheit derart ausgelegt ist, dass sie mittels einer externen und/oder internen Spannungsquelle mit Energie versorgt wird.

Ein Anschluss einer Spannungsquelle an die Vorrichtung zur Ermittlung einer persönlichen Tinnitus-Frequenz kann beispielsweise über ein Element, welches zum Beispiel als ein Mini-USB-Anschluss, ein induktiver Ladeanschluss, oder ein herkömmlicher Netzstecker-Anschluss ausgebildet ist, erfolgen.

Außerdem ist es denkbar, dass eine Batterie oder ein Akku, welcher an der Vorrichtung zur Ermittlung einer persönlichen Tinnitus-Frequenz angeordnet ist, die Vorrichtung mit Energie beliefert. Beispielsweise kann über eine mit der Vorrichtung zur Ermittlung einer persönlichen Tinnitus-Frequenz angeordnete externe Spannungsquelle eine Batterie bzw. ein Akku, welcher an der Vorrichtung angeordnet ist, geladen werden.

Vorteilhaft kann sein, dass an die Audiosignal-Erzeugereinheit eine Anzeigeeinheit angeordnet ist.

Eine Anzeigeeinheit, beispielsweise ein Display, kann insbesondere eine einstellbare Tinnitus-Frequenz und/oder eine Lautstärke, beziehungsweise eine Amplitude der einstellbaren Tinnitus-Frequenz und/oder eine Temperatur und/oder einen zweiten Audiosignal-Modus und/oder eine Bezeichnung einer Audiodatei eines ersten und/oder zweiten Audiosignals anzeigen.

Die Anzeigeeinheit kann zum Beispiel mehrzellig und/oder mehrfarbig ausgestaltet sein.

Außerdem kann die Anzeigeeinheit zum Beispiel einen Ladezustand eines angeordneten Energiespeichers, beispielsweise einer Batterie oder eines Akkus, anzeigen.

Vorteilhafterweise ist an die Audiosignal-Erzeugereinheit ein Element für einen Audio-Kopfhörer für sowohl ein erstes als auch ein zweites Audiosignal angeordnet.

Es ist beispielsweise vorstellbar, dass an der Vorrichtung zur Ermittlung einer persönlichen Tinnitus-Frequenz zwei Audiosignal-Kopfhörer gleichzeitig angeordnet sind.

Vorstellbar ist beispielsweise, dass an der Vorrichtung drei Kontaktmittel angeordnet sind. Ein erstes Kontaktmittel an welchem ein erster Audiokopfhörer angeordnet ist, welcher das erste Audiosignal bereitstellt, und ein zweites Kontaktmittel an welchem ein zweiter Audiokopfhörer angeordnet ist, welcher das zweite Audiosignal bereitstellt. Zusätzlich kann ein drittes Kontaktmittel an der Vorrichtung angeordnet sein, an welchen zum Beispiel ein Kabel angeordnet werden kann, welches mit einer Heizanordnung am Kopfhörer verbunden ist und mittels welchem eine Spannung für die Heizanordnung bereitgestellt wird.

Denkbar ist auch, dass an der Vorrichtung zum Beispiel ein erstes Kontaktmittel angeordnet ist, an welchem ein einziger Audiokopfhörer angeordnet ist, welcher sowohl das erste Audiosignal als auch das zweite Audiosignal bereitstellt. Außerdem kann ein zweites Kontaktmittel an der Vorrichtung angeordnet sein, an welches ein mit der Heizanordnung am Kopfhörer verbundenes Kabel angeordnet werden kann und mittels welchem die Spannung für die Heizanordnung bereitgestellt wird.

Außerdem vorteilhaft ist, dass an der Vorrichtung beispielsweise ein einziges Kontaktmittel angeordnet ist, an welchem ein einziger Audiokopfhörer angeordnet ist. Sowohl das erste Audiosignal als auch das zweite Audiosignal und die Spannung für die Heizanordnung am Kopfhörer kann beispielsweise mittels des Kontaktmittels bereitgestellt werden.

Insbesondere denkbar ist, dass an der Vorrichtung genau zwei Kontaktmittel angeordnet sind. Ein erstes Kontaktmittel an welchem ein erster Audiokopfhörer angeordnet ist, welcher das erste Audiosignal bereitstellt und ein zweites Kontaktmittel an welchem ein zweiter Audiokopfhörer angeordnet ist, welcher das zweite Audiosignal bereitstellt und an welchem eine Heizanordnung angeordnet ist.

Ein erster Audiosignal-Kopfhörer kann als Ohrstöpsel ausgebildet sein, mittels welchem ein zweites Audiosignal mit einer veränderten persönlichen Tinnitus-Frequenz bereitgestellt wird und welcher beispielsweise auch zur Erwärmung des Ohrs dienen kann.

Ein zweiter Audiosignal-Kopfhörer kann über dem Ohr bzw. der Ohrmuschel und dem am Ohr angeordneten zweiten Audiosignal-Kopfhörer angeordnet sein und Kompensationsmittel zur Geräuschunterdrückung von Umweltgeräusche beinhalten.

Ein Nutzer der Vorrichtung zur Ermittlung einer persönlichen Tinnitus-Frequenz kann beispielsweise zunächst einen ersten Kopfhörer, welcher das erste Audiosignal bereitstellt und als Ohrstöpsel ausgebildet und insbesondere beheizbar sein kann, am Ohr anordnen. Im Weiteren kann ein Nutzer zusätzlich zum ersten Kopfhörer einen zweiten Kopfhörer, welcher das zweite Audiosignal bereitstellt und zum Beispiel als ein die Ohrmuschel umschließender Kopfhörer ausgebildet ist und welcher Kompensationsmittel zur aktiven Geräuschunterdrückung umfasst, am Ohr anordnen.

Eine kombinierte Verwendung der beiden Audiosignal-Kopfhörer ermöglicht eine vergleichsweise bessere Bestimmung der persönlichen Tinnitus-Frequenz und/oder eine vergleichsweise bessere, beziehungsweise angenehmere Wellness-Behandlung durch das eingespielte zweite Audiosignal und zusätzlich zum Beispiel einer Erwärmung einer Ohrregion.

Auch ist es von Vorteil, dass die Audiosignal-Erzeugereinheit als mobile Einheit ausgebildet ist.

Die Audiosignal-Erzeugereinheit kann beispielsweise mit einem internen Energiespeicher ausgestattet sein und in der Größe derart ausgestaltet sein, dass die Audiosignal-Erzeugereinheit vergleichsweise bequem am Körper des Nutzers angeordnet werden kann. Hierdurch kann eine hohe Bewegungsfreiheit des Nutzers ermöglicht werden. Beispielsweise kann der Nutzer die Vorrichtung zur Ermittlung einer persönlichen Tinnitus-Frequenz in häuslicher Umgebung nutzen und dabei beispielsweise häuslichen Tätigkeiten nachgehen.

In einer weiteren vorteilhaften Modifikation ist an der Audiosignal-Erzeugereinheit ein Element für ein Detektionselement vorgesehen.

Das Detektionselement kann beispielsweise als Messelement, welches auf einem, optischen Messverfahren basiert, oder als Messelement, welches auf einem Messverfahren mittels Ultraschall basiert, ausgestaltet sein.

Mittels des Detektionselementes kann eine Körperfunktion des Nutzers der Vorrichtung zur Ermittlung einer persönlichen Tinnitus-Frequenz, wie zum Beispiel der Puls und/oder der Hautwiderstand und/oder der Blutdruck und/oder die Atmungsfrequenz, bestimmt werden.

Hierdurch kann vorteilhaft ein Feedback des Nutzers auf das eingespielte zweite Audiosignal ermittelt werden.

Das Detektionselement kann vorzugsweise mittels eines Kabels oder über eine Funkverbindung an einem Kontaktmittel an der Audiosignal-Erzeugereinheit angeordnet sein.

Vorteilhafterweise kann während einer Therapierung eines Tinnitus eines Nutzers mittels des zweiten Audiosignals, der Modus des zweiten Audiosignals, basierend auf den ermittelten Körperfunktionen über ein an der Audiosignal-Erzeugereinheit angeordnetes Regelelement, insbesondere automatisch, geregelt werden. Außerdem vorstellbar ist, dass das Regelelement den Modus des zweiten Audiosignals basierend auf einer Änderung der Körperfunktion regelt. Beispielsweise kann die Frequenz und/oder die Phase und/oder die Amplitude der ermittelten persönlichen Tinnitus-Frequenz und/oder der Modus im zweiten Audiosignal mittels eines Regelelements basierend auf einer ermittelten Körperfunktion geregelt werden.

### Beschreibung der Ausführungsbeispiele

Anhand von nachstehenden schematischen Zeichnungen werden mehrere Ausführungsbeispiele unter Angabe weiterer Einzelheiten und Vorteile näher erläutert. Es zeigen:
- Figur 1: eine schematische Darstellung einer erfindungsgemäßen Vorrichtung,
- Figur 2: ein schematischer Ablauf einer Therapieeinheit mittels der erfindungsgemäßen Vorrichtung,
- Figur 3: eine weitere schematische Darstellung einer Audiosignal-Erzeugereinheit der erfindungsgemäßen Vorrichtung und
- Figur 4: eine schematische Darstellung einer Anordnung der erfindungsgemäßen Vorrichtung an einem Nutzer.

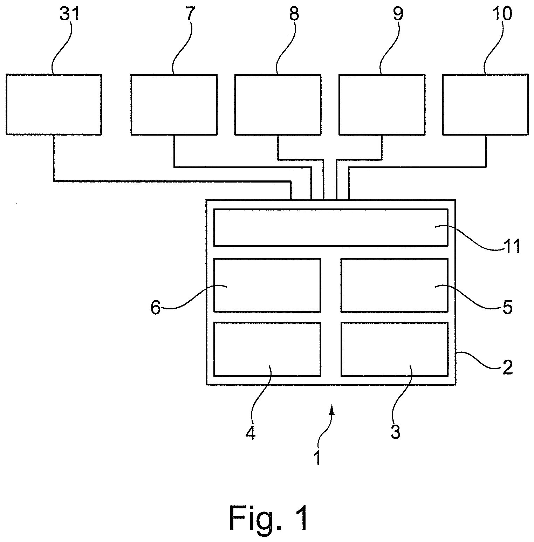

In Figur 1 ist eine erfindungsgemäße Vorrichtung 1 dargestellt, bestehend aus einer Audiosignal-Erzeugereinheit 2 und daran angeordnet Kopfhörer 7, 8, ein Netzstecker 9, eine Speichereinheit 10 und ein Detektionselement 31. Die Audiosignal-Erzeugereinheit 2 umfasst eine Anzeigeeinheit 3, Einstellmittel 4, ein Regelelement 5 zur Einstellung von Audiosignalen, eine interne Energiequelle 6 und Kontaktmittel 11 für die externen Kopfhörer 7, 8, den Netzstecker 9, die Speichereinheit 10 und das Detektionselement 31.

Die erfindungsgemäße Vorrichtung 1 muss nicht alle Elemente enthalten und nicht in der dargestellten integrierten bzw. externen Ausgestaltung ausgebildet sein.

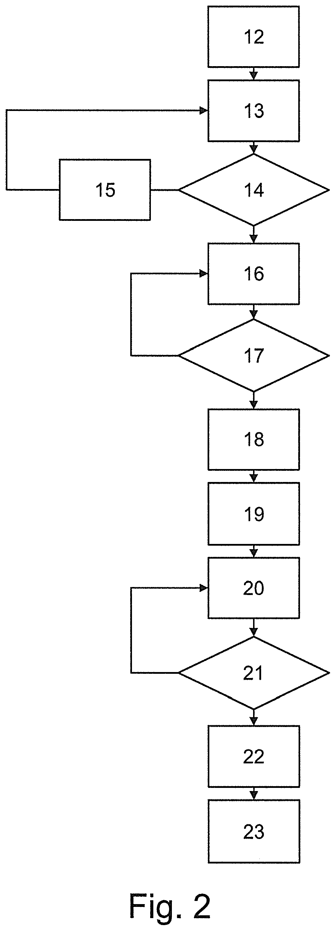

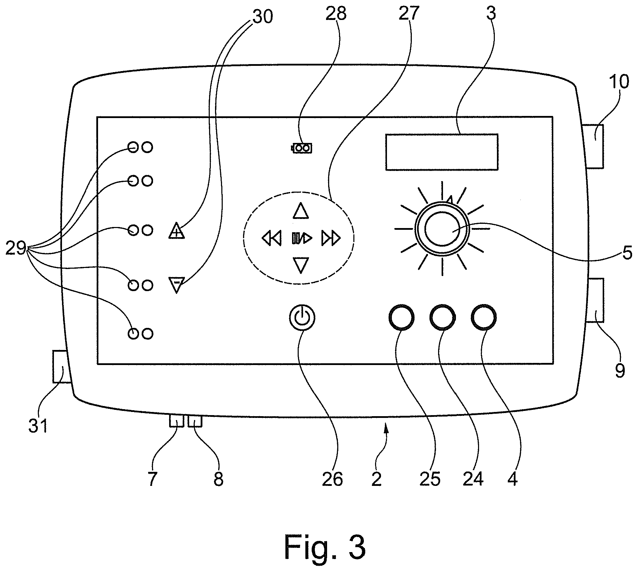

Eine Verwendung der Vorrichtung zur Ermittlung einer persönlichen Tinnitus-Frequenz kann beispielsweise wie in Figur 2 dargestellt ablaufen (siehe hierzu auch Figur 1 und Figur 3).

In einem ersten Schritt 12 aktiviert der Nutzer 33 die Audiosignal-Erzeugereinheit, indem er einen Ein-/Ausschalter 26 an der Audiosignal-Erzeugereinheit 2 betätigt. Gegebenenfalls schließt der Nutzer 33 an der Vorrichtung 1 außerdem eine Speichereinheit 10 an.

In einem nächsten Schritt 13 betrachtet der Nutzer 33, den Ladezustand einer Batterie oder eines Akku der Vorrichtung. Diesen kann er beispielsweise der Anzeigeeinheit 3 an der Vorrichtung 1 entnehmen.

Sollte sich bei der Betrachtung im weiteren Schritt 14 ergeben, dass der Ladezustand der Vorrichtung 1 nicht ausreichend für eine Verwendung durch den Nutzer 33 ist, hat der Nutzer 33 die Möglichkeit, zum einen in einem weiteren Schritt 15 das Gerät an den Netzstecker 9 anzuschließen und das Gerät damit zu laden und so lange zu warten, bis die Vorrichtung 1 einen ausreichenden Ladezustand aufweist, um eine Verwendung der Vorrichtung 1 über die volle Nutzungsdauer zu gewährleisten, oder zum anderen den Netzstecker 9 zum Laden der internen Energiequelle 6 anzuschließen und gleichzeitig mit der Verwendung der Vorrichtung ortsgebunden, durch den Netzstecker 9, fortzufahren.

Ist der Ladezustand der Vorrichtung 1 ausreichend, setzt der Nutzer 33 in einem weiteren Schritt 16 einen Kopfhörer mit aktiver Geräuschunterdrückung 7 auf und ermittelt anschließend ungestört von Umweltgeräuschen seine persönliche Tinnitus-Frequenz.

In einem folgenden Schritt 17 prüft der Nutzer 33 das Ergebnis der Ermittlung und wiederholt gegebenenfalls Schritt 16, bis die persönliche Tinnitus-Frequenz korrekt ermittelt wurde.

In einem anschließenden Schritt 18 tauscht der Nutzer 33 die Kopfhörer mit aktiver Geräuschunterdrückung 7 gegen beheizbare In-Ear-Kopfhörer 8.

In einem nächsten Schritt 19 aktiviert der Nutzer 33 die Heizung der In-Ear-Kopfhörer 8 zur Erwärmung des Ohrs und wählt ein zweites Audiosignal über Einstellmittel 4 an der Audiosignal-Erzeugereinheit 2 aus und aktiviert es.

In einem folgenden Schritt 20 verändert der Nutzer 33 gegebenenfalls sowohl die Lautstärke des zweiten Audiosignals, über die Einstellmittel 4 an der Audiosignal-Erzeugereinheit 2 als auch die Temperatur mittels des Einstellmittels 4 an der Audiosignal-Erzeugereinheit 2.

Sollte eine Prüfung im Schritt 21 ergeben, dass die Lautstärke und/oder die Temperatur für den Nutzer 3 nicht anwendbar ist, wiederholt der Nutzer 33 Schritt 20.

Sind die Lautstärke und die Temperatur für den Nutzer 33 angenehm, wählt der Nutzer 33 in einem weiteren Schritt 22, beispielsweise über Einstellmittel 4, aus, in welchem Modus die Musik bzw. das zweite Audiosignal an der Audiosignal-Erzeugereinheit 2 aktiviert werden soll. Beispielsweise könnte der Nutzer 33 ein Modus auswählen, in welchem die Tinnitus-Frequenz im zweiten Audiosignal in einem schmalbandigen Bereich um die persönliche Tinnitus-Frequenz ausblendet ist. Er könnte vorzugsweise einen Modus wählen, in dem zum Beispiel die Phase der Tinnitus-Frequenz im zweiten Audiosignal positiv (konstruktiv) oder negativ (destruktiv) an der Stelle oder im Bereich der persönlichen Tinnitus-Frequenz überlagert ist.

In einem abschließenden Schritt 23 startet der Nutzer 33 über die Einstellmittel 4 eine Tinnitus-Therapie-Einheit.

Figur 3 zeigt eine schematische Darstellung der Audiosignal-Erzeugereinheit 2. Die Audiosignal-Erzeugereinheit 2 kann ein Einstellmittel 24 umfassen, mit welchem die Lautstärke des ersten oder zweiten Audiosignals einstellbar ist. Weiterhin kann die Audiosignal-Erzeugereinheit 2 ein Einstellmittel 25 umfassen, mit welchem der Modus der Tinnitus-Behandlung einstellbar ist. Außerdem ist an der Audiosignal-Erzeugereinheit 2 ein Schalter 26 angeordnet, mit welchem die Vorrichtung 1 ein- bzw. ausgeschaltet werden kann.

Zur Auswahl des ersten bzw. des zweiten Audiosignals stehen Bedienelemente 27 an der Audiosignal-Erzeugereinheit 2 dem Nutzer 33 zur Verfügung. Mit diesen kann der Nutzer 33 vergleichsweise bequem die entsprechende Audiodatei auswählen. Außerdem kann die Audiosignal-Erzeugereinheit 2 ein Anzeigeelement 28 umfassen, welches den Ladezustand der externen oder internen Energiequelle, beispielsweise des intern angeordneten Akkus, darstellt.

Darüber hinaus können an der Audiosignal-Erzeugereinheit 2 Anzeigeelemente 29 angeordnet sein, mit welchen die Amplitude bzw. die Phase der persönlichen Tinnitus-Frequenz für den Nutzer 33 visualisiert werden. Mittels Bedienelemente 30 kann der Nutzer 33 beispielsweise die Amplitude bzw. Phase variieren.

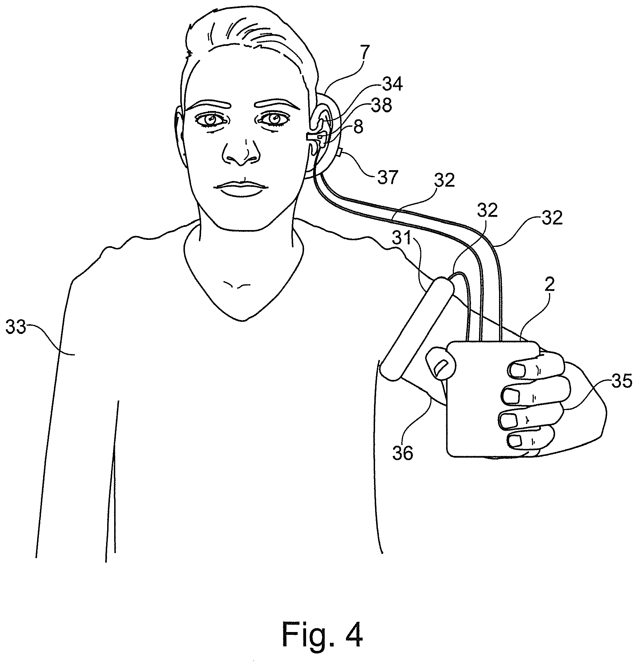

Figur 4 zeigt die Anordnung der erfindungsgemäßen Vorrichtung an einem Nutzer 33. An einem Ohr 34 des Nutzers 33 ist ein als Ohrstöpsel ausgebildeter Kopfhörer 8 angeordnet. An diesen kann beispielsweise eine Heizanordnung 38 angeordnet sein. Weiterhin kann dieser zum Beispiel das zweite Audiosignal wiedergeben. Außerdem ist über dem Ohr 34 und über dem Kopfhörer 8 ein Kopfhörer 7 angeordnet, der derart ausgebildet ist, dass er das Ohr 34 vollständig umschließt. Am Kopfhörer 7 können beispielsweise Kompensationsmittel 37 zur aktiven Geräuschunterdrückung von Umweltgeräuschen angeordnet sein.

Mit der Audioerzeugereinheit 2 sind die Kopfhörer 7, 8 mittels Kabel 32 verbunden. Die Audioerzeugereinheit 2 ist insbesondere derart ausgebildet, dass sie als mobiles Gerät mit einer Hand 35 durch den Nutzer 33 gehalten werden kann.

Am Arm 36 des Nutzers 33 ist zum Beispiel außerdem ein Detektionselement 31 angeordnet, mit welchem beispielsweise der Puls oder der Blutdruck des Nutzers 33 ermittelt werden kann. Das Detektionselement 31 ist zum Beispiel mittels eines Kabels 32 mit der Audioerzeugereinheit 2 verbunden.

### Bezugszeichenliste:

- 1: Vorrichtung
- 2: Audiosignal-Erzeugereinheit
- 3: Anzeigeeinheit
- 4: Einstellmittel
- 5: Regelelement
- 6: Energiequelle
- 7: Kopfhörer
- 8: Kopfhörer
- 9: Netzstecker
- 10: Speichereinheit
- 11: Kontaktmittel
- 12 - 23: Nutzungsschritt
- 24: Einstellmittel
- 25: Einstellmittel
- 26: Schalter
- 27: Bedienelement
- 28: Anzeigeelement
- 29: Anzeigeelement
- 30: Bedienelement
- 31: Detektionselement
- 32: Kabel
- 33: Nutzer
- 34: Ohr
- 35: Hand
- 36: Arm
- 37: Kompensationsmittel
- 38: Heizanordnung

## Patentansprüche

1. Vorrichtung (1) zur Ermittlung einer persönlichen Tinnitus-Frequenz bestehend aus einer Audiosignal-Erzeugereinheit (2) und einem zugeordneten Audiosignal-Kopfhörer (8), welche ein in der Frequenz stufenlos einstellbares erstes Audiosignal bereitstellt, **dadurch gekennzeichnet, dass** der Audiosignal-Kopfhörer (8) als ein In-Ear-Kopfhörer ausgebildet ist und eine Heizanordnung (38) zur Erwärmung eines Ohrbereichs am Audiosignal-Kopfhörer (8) angeordnet ist, wobei an der Vorrichtung (1) ein Einstellmittel zur Einstellung einer Temperatur des Audiosignal-Kopfhöres angeordnet ist.

2. Vorrichtung (1) nach dem vorangegangen Anspruch, **dadurch gekennzeichnet, dass** Kompensationsmittel (37) vorgesehen sind um Umweltgeräusche durch eine aktive Geräuschunterdrückung zu eliminieren.

3. Vorrichtung (1) nach einem vorangegangen Anspruch **dadurch gekennzeichnet, dass** über ein an die Audiosignal-Erzeugereinheit angeordnetes Element (4) ein erstes und/oder zweites Audiosignal auswählbar ist.

4. Vorrichtung (1) nach einem vorangegangen Anspruch **dadurch gekennzeichnet, dass** die Vorrichtung ein Element (4) aufweist, mit welchem ein Nutzer (33) eine persönliche Tinnitus-Frequenz für jedes Ohr getrennt ermitteln kann.

5. Vorrichtung (1) nach einem vorangegangen Anspruch **dadurch gekennzeichnet, dass** die Vorrichtung ein Element (4) aufweist, mit welchem eine ermittelte persönliche Tinnitus-Frequenz in einem schmalen Frequenzbereich stufenlos regelbar verändert werden kann.

6. Vorrichtung (1) nach einem vorangegangen Anspruch **dadurch gekennzeichnet, dass** die Vorrichtung dazu ausgebildet ist, ein zweites Audiosignal zu erzeugen, in welchem eine ermittelte persönliche Tinnitus-Frequenz verändert ist.

7. Vorrichtung (1) nach einem vorangegangen Anspruch **dadurch gekennzeichnet, dass** an die Audiosignal-Erzeugereinheit (2) ein Element (11) angeordnet ist, an welches eine Speichereinheit angeschlossen werden kann.

8. Vorrichtung (1) nach einem vorangegangen Anspruch **dadurch gekennzeichnet, dass** die Audiosignal-Erzeugereinheit (2) derart ausgelegt ist, dass sie mittels einer externen und/oder internen Spannungsquelle (6,9) mit Energie versorgt wird.

9. Vorrichtung (1) nach einem vorangegangen Anspruch **dadurch gekennzeichnet, dass** ein Kontaktmittel (11) an der Audiosignal-Erzeugereinheit (2) für einen Audio-Kopfhörer (7,8) für sowohl ein erstes als auch ein zweites Audiosignal angeordnet ist.

10. Vorrichtung (1) nach einem vorangegangen Anspruch **dadurch gekennzeichnet, dass** die Audiosignal-Erzeugereinheit (2) als mobile Einheit ausgebildet ist.

## Claims

1. Device (1) for determining a personal tinnitus frequency consisting of an audio signal generator (2) and an assigned audio signal earphone (8), which provides a first audio signal which can be continuously adjusted in frequency, **characterised in that** the audio signal earphone (8) is designed as an in-ear earphone and a heating arrangement (38) for heating an area of the ear is arranged on the audio signal earphone (8), wherein on the device (1) an adjusting means is arranged for adjusting a temperature of the audio signal earphone.

2. Device (1) according to the preceding claim, **characterised in that** compensation means (37) are provided in order to eliminate environmental sounds by means of active noise suppression.

3. Device (1) according to any preceding claim, **characterised in that** a first and/or second audio signal can be selected via an element (4) arranged on the audio signal generator.

4. Device (1) according to any preceding claim, **characterised in that** the device has an element (4) by means of which a user (33) can separately determine a personal tinnitus frequency for each ear.

5. Device (1) according to any preceding claim, **characterised in that** the device has an element (4), by means of which a determined personal tinnitus frequency can be adjusted continuously in a narrow frequency range.

6. Device (1) according to any preceding claim, **characterised in that** the device is designed to generate a second audio signal, in which a determined personal tinnitus frequency is adjusted.

7. Device (1) according to any preceding claim, **characterised in that** an element (11) is arranged on the audio signal generator (2), to which element a memory unit can be connected.

8. Device (1) according to any preceding claim, **characterised in that** the audio signal generator (2) is configured such that it is provided with power by means of an external and/or internal voltage source (6, 9).

9. Device (1) according to any preceding claim, **characterised in that** a contact means (11) is arranged on the audio signal generator (2) for an audio earphone (7, 8) for both a first and also a second audio signal.

10. Device (1) according to any preceding claim, **characterised in that** the audio signal generator (2) is designed as a mobile unit.

## Revendications

1. Dispositif (1) pour déterminer une fréquence d'acouphène personnelle, composé d'une unité pour générer un signal audio (2) et d'un casque de signal audio (8) associé qui fournit un premier signal audio dont la fréquence peut être ajustée en continu, **caractérisé en ce que** le casque de signal audio (8) est conçu comme un écouteur intra-auriculaire et qu'un dispositif de chauffage (38) est disposé sur le casque de signal audio (8) pour chauffer une région de l'oreille, des moyens de réglage étant disposés sur le dispositif (1) pour régler une température du casque de signal audio.

2. Dispositif (1) selon la revendication précédente, **caractérisé en ce que** des moyens de compensation (37) sont prévus pour éliminer les bruits de l'environnement par suppression active du bruit.

3. Dispositif (1) selon l'une des revendications précédentes, **caractérisé en ce qu'**un premier et/ou un deuxième signal audio peuvent être sélectionnés par le biais d'un élément (4) disposé sur l'unité pour générer un signal audio.

4. Dispositif (1) selon l'une des revendications précédentes, **caractérisé en ce que** le dispositif comprend un élément (4) avec lequel un utilisateur (33) peut déterminer une fréquence d'acouphène personnelle pour chaque oreille séparément.

5. Dispositif (1) selon l'une des revendications précédentes, **caractérisé en ce que** le dispositif comprend un élément (4) avec lequel une fréquence d'acouphène personnelle déterminée peut être modifiée par un réglage en continu dans une plage de fréquences étroite.

6. Dispositif (1) selon l'une des revendications précédentes, **caractérisé en ce que** le dispositif est conçu pour générer un deuxième signal audio dans lequel une fréquence d'acouphène personnelle déterminée est modifiée.

7. Dispositif (1) selon l'une des revendications précédentes, **caractérisé en ce qu'**un élément (11) est disposé sur l'unité pour générer un signal audio (2) et auquel une unité de mémoire peut être connectée.

8. Dispositif (1) selon l'une des revendications précédentes, **caractérisé en ce que** l'unité pour générer un signal audio (2) est conçue de sorte à être alimentée en énergie au moyen d'une source de tension externe et/ou interne (6, 9).

9. Dispositif (1) selon l'une des revendications précédentes, **caractérisé en ce que** des moyens de contact (11) pour un casque audio (7, 8) sont disposés sur l'unité pour générer un signal audio (2) aussi bien pour un premier que pour un deuxième signal audio.

10. Dispositif (1) selon l'une des revendications précédentes, **caractérisé en ce que** l'unité pour générer un signal audio (2) est conçue sous la forme d'une unité mobile.
